(19) Europäisches Patentamt
European Patent Office
Office européen des brevets

(11) Veröffentlichungsnummer: **0 053 818**
**B1**

# (12) EUROPÄISCHE PATENTSCHRIFT

(45) Veröffentlichungstag der Patentschrift: **08.05.85**

(51) Int. Cl.⁴: **C 07 C 85/06**, C 07 C 85/24

(21) Anmeldenummer: **81110159.1**

(22) Anmeldetag: **04.12.81**

## (54) Verfahren zur Herstellung von Cyclohexylaminen durch Hydrierung von aromatischen Aminen.

(30) Priorität: **04.12.80 DE 3045719**

(43) Veröffentlichungstag der Anmeldung: **16.06.82 Patentblatt 82/24**

(45) Bekanntmachung des Hinweises auf die Patenterteilung: **08.05.85 Patentblatt 85/19**

(84) Benannte Vertragsstaaten: **AT CH DE FR GB IT LI NL SE**

(56) Entgegenhaltungen:
**CH - A - 445 476**
**FR - A - 1 506 010**
**FR - A - 1 530 477**
**US - A - 3 196 179**

(73) Patentinhaber: **BASF Aktiengesellschaft, Carl-Bosch-Strasse 38, D-6700 Ludwigshafen (DE)**

(72) Erfinder: **Goetz, Norbert, Dr., Schoefferstrasse 25, D-6520 Worms (DE)**
Erfinder: **Jacobs, Peter, Dr., Kalmitstrasse 2, D-6718 Gruenstadt (DE)**
Erfinder: **Hupfer, Leopold, Dr., Waltershoehe 3, D-6701 Friedelsheim (DE)**
Erfinder: **Toussaint, Herbert, Dr., Knietschstrasse 11, D-6710 Frankenthal (DE)**
Erfinder: **Reiss, Wolfgang, Dr., Bannwasserstrasse 70, D-6700 Ludwigshafen (DE)**

EP 0 053 818 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die vorliegende Erfindung betrifft ein Verfahren zur Herstellung von Cyclohexylaminen durch Hydrierung von aromatischen Aminen in Gegenwart von Palladiumkatalysatoren.

Die Hydrierung von aromatischen Aminen zu cycloaliphatischen Aminen in Gegenwart von auf Trägern befindlichen Edelmetallkatalysatoren ist vielfach beschrieben, wobei in der Mehrzahl Ruthenium-Katalysatoren (J. Org. Chem. 27 (1962) S. 3568 bis 3572, DE 27 45 172, DE 25 02 894, US 3 697 449, US 3 644 522, DE 21 32 547, US 3 404 098, DE 21 25 790, JP 70 30 696, NL 6 709 040), in geringerem Maße Rhodium-Katalysatoren (Bl. Chem. Soc. Japan 41 (1968), S. 2194 bis 2195, BE 739 376, JP 70 19 901, JP 72 35 424) verwendet werden. In einigen wenigen Fällen wird für die obengenannte Umsetzung auch die Verwendung von Palladium-Katalysatoren in Gegenwart von Säuren (US 3 520 928) oder auf ein spezielles Verfahren Atmosphärendruck unter ständiger Entfernung der cycloaliphatischen Amine aus der Reaktionszone (US 3 117 992) verwiesen. Nach dem Vorschlag der FR-A-1 530 477 soll Anilin an einem einfachen Palladium-Trägerkatalysator bei leicht erhöhtem Druck (4 bar) in Cyclohexylamin übergeführt werden, dabei entsteht aber Dicyclohexylamin in beträchtlichen Mengen als Nebenprodukt.

Weiterhin sollen speziell bei der Hydrierung des unsubstituierten Anilins zum Cyclohexylamin in bezug auf Aktivität und Nebenproduktbildung (Bildung von Dicyclohexylamin, N-Phenylcyclohexylamin) Rhodium- und Rhuteniumkatalysatoren wesentlich besser wirkend sind als Palladiumkatalysatoren (J. Org. Chem. 29 (1964), S. 3082 bis 3084, J. Chem. Soc. Japan 90 (1969) S. 91 bis 95.

Es wurde nun gefunden, daß man cycloaliphatische Amine der allgemeinen Formel I

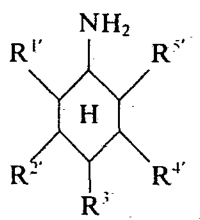

(I)

durch Hydrierung von entsprechenden aromatischen Aminen der allgemeinen Formel II

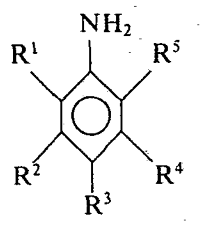

(II)

in guter Ausbeute und Reinheit erhält, wenn man die Umsetzung bei 100 bis 300°C, vorzugsweise bei 150 bis 250°C und einem Druck von wenigstens 100 bar, vorzugsweise bei 150 bis 300 bar, in Gegenwart von Ammoniak und eines Trägerkatalysators durchführt, der als Träger Aluminiumoxid oder eine Aluminiumoxid enthaltende Verbindung, Palladium und eine basische Verbindung und/oder ein Element der Gruppe 1 B, 2 B, 7 B, Eisen, Kobalt oder Nickel enthält.

Nach dem erfindungsgemäßen Verfahren werden die gesuchten Cyclohexylamine bei vollständigem Umsatz und hoher Ausbeute frei von Nebenprodukten wie Dicyclohexylaminen, Phenylcyclohexylaminen oder Diphenylaminen gewonnen. Weiterhin besitzen die erfindungsgemäßen Palladiumkatalysatoren im Vergleich zu Ruthenium- und Rhodiumkatalysatoren den Vorteil, daß sie vielseitig verwendbar sind und damit wesentlich den Aufbau von Hydrieranlagen ermöglichen, die für verschiedene Zwecke geeignet sind. Vorteilhaft ist dabei die lange Lebensdauer der verwendeten Palladiumkatalysatoren; so ist beispielsweise nach einer Dauerbeanspruchung von über 1200 Betriebsstunden kein Aktivitätsabfall zu verzeichnen.

Aliphatische Gruppen mit 1 bis 20 Kohlenstoffatomen, cycloaliphatische Gruppen mit 5 bis 20 Kohlenstoffatomen, Arylgruppen mit 6 bis 20 Kohlenstoffatomen oder Aralkyl- oder Alkylarylgruppen mit 7 bis 20 Kohlenstoffatomen sind Beispiele für Substituenten R. Diese können gegebenenfalls Sauerstoff und/oder Stickstoff als Heteroatome in der Kette enthalten oder es können zwei Substituenten durch eine Molekülbrücke miteinander verbunden sein. Wenn ein Substituent dem ohne ihn verbleibenden Molekülrest entspricht, bedeutet die Formel I ein mehrkerniges, insbesondere zweikerniges Polyamin (Diamin).

$R^1$ bis $R^5$ können gleiche oder verschiedene Substituenten und selbstverständlich Wasserstoff bedeuten, wobei Alkyl- oder Aralkylreste mit jeweils 1 bis 20 C-Atomen Kettenlänge besonders wichtige Substituenten darstellen. Benachbarte Substituenten können zusammen mit dem Rest einen Ring bilden, so daß als aromatische Amine i. S. der Erfindung auch Naphthylamine und teilweise hydrierte Naphthylamine zu gelten haben. Wenn ein Substituent hydrierbar ist, geht er bei der Umsetzung in die

wasserstoff-reichere Form über.

Zwar ist die Umsetzung von Anilin selbst weniger wichtig, aber sie ist technisch möglich. Andere, substituierte Aniline sind etwa o-, m- und p-Toluidin, o-Ethylanilin, o-n-Butylanilin, o-sek.-Butylanilin, 2,4-Dimethylanilin, 2,6-Dimethylanilin, 2,3,6-Trimethylanilin, 2,4,6-Trimethylanilin, 2-Cyclohexylanilin, 2,6-Dimethyl-3-phenyl-anilin, 2,6-Diethylanilin, 2,5-Diisopropylanilin, 2,6-Di-tert.-butylanilin, 2-Methyl-6-sek.-butylanilin, 3-tert.-Butylanilin, $\alpha$-Naphthylamin, $\beta$-Naphthylamin, 4,4'-Diaminodiphenylmethan.

Der erforderliche Ammoniak kann — bezogen auf das eingesetzte aromatische Amin — in stöchiometrischer Menge oder in einem vorzugsweise 2- bis 10fachen oder auch höheren Überschuß angewendet werden.

Das Verfahren kann kontinuierlich (fortlaufend) oder diskontinuierlich (absatzweise) durchgeführt werden. In jedem Falle kann der Katalysator in einem Festbett oder als Suspension angeordnet sein. Bevorzugt bei kontinuierlicher Arbeitsweise ist ein fest angeordneter Katalysator. Bei kontinuierlicher Arbeitsweise wird der Ammoniak zusammen mit dem Wasserstoff über den Katalysator geleitet. Im Falle diskontinuierlichen Betriebs führt man so lange Wasserstoff zu, bis das eingesetzte aromatische Amin vollständig umgesetzt ist. Die Umsetzung kann ohne Lösungsmittel oder in Anwesenheit von Lösungsmitteln, die unter den Reaktionsbedingungen chemisch indifferent sind, durchgeführt werden.

Als Lösungsmittel kommen beispielsweise in Frage: Methanol, Ethanol, n-Butanol, Tetrahydrofuran, Dioxan, Cyclohexylmethylether, Methylglykol, Ethylglykol, 1,2-Dimethoxyethan, 2,6-Dimethylcyclohexylamin, N-Methylpiperidin, N-Ethylpiperidin, N-Methylmorpholin, N-Methylpyrrolidin, Cyclohexan. In vielen Fällen ist das Zielprodukt selbst das günstigste Lösungsmittel; man führt einen Teilstrom zurück, wenn eine entsprechende Verdünnung gewünscht wird.

Der Katalysator enthält Palladium auf einem Träger aus Aluminiumoxid oder einem seiner Umsetzungsprodukte, wie sich aus dem folgenden im einzelnen ergibt. Der Katalysator enthält nämlich Elemente bzw. Verbindungen von Elementen der Gruppen 1 B, 2 B und 7 B des Periodensystems sowie Eisen, Kobalt und Nickel oder basische Stoffe, d. h. Verbindungen der ersten bis dritten Hauptgruppe des Periodensystems. Die letzteren, basischen Stoffe bilden vorzugsweise einen Bestandteil des Trägers. Natürlich können die Zusätze allein oder im Gemisch miteinander neben dem Edelmetall vorliegen. Der Träger besteht aus Aluminiumoxid oder einem unter Mitwirkung von Aluminiumoxid aufgebauten Stoff. Aluminiumsilikat oder Spinelle des Aluminiums sind solche Stoffe. Die Zusätze, die dem Katalysator seine Eigenschaften verleihen, können sich an der Oberfläche des Katalysators befinden, zusammen mit dem übrigen Aluminiumoxid usw. in Form einer Mischung den Träger darstellen oder mit dem ursprünglichen Träger durch nachträgliches Tempern ein gemeinsames Kristallgitter bilden (beispielsweise bei Aluminiumoxidträgern Bildung von typischen Spinellgittern mit bestimmten Metallen). Das bedeutet, daß der Träger Aktivität und auch Lebensdauer des Katalysators günstig beeinflussen kann.

Als Zusätze kommen in Frage:

a) Basische Zuätze, wie Oxide, Hydroxide oder Carbonate der Alkalimetalle, vorzugsweise des Lithiums und Natriums, der Erdalkalimetalle, vorzugsweise des Magnesiums und Calciums und der Seltenerdmetalle, vorzugsweise des Cers und Praseodyms (Neodyms).
b) Weitere Metalle, wie Eisen, Nickel, Kobalt, Mangan, Zink, Cadmium und Silber.

Die Zusätze können gemeinsam mit dem Palladium durch Tränken des Trägermaterials mit Lösungen von z. B. Nitraten, Chloriden, Formiaten oder Oxalaten aufgebracht werden. Durch anschließende thermische Behandlung, die gewöhnlich zwischen 400 und 600°C durchgeführt wird, erfolgt Überführung in die Oxidform. Soll bei Aluminiumoxidträgern mit geeigneten Metallen (Mg, Zn, Co, Mn, Li) Spinellbildung erzielt werden, muß nach der Tränkung auf eine Temperatur von 900—1300°C erhitzt werden (siehe Ullmanns Encyklopädie der technischen Chemie, 3. Auflage (1955) Band 6, S. 242—244, Gmelin, System-Nr. 35, Al, Tl, 1934—1935, S. 26—28) und anschließend in der üblichen Weise das Edelmetall aufgebracht werden.

Manche Zusätze, wie beispielsweise Calciumoxid oder Magnesiumoxid lassen sich mit einem Träger wie Aluminiumoxid mischen und bilden dann nach gemeinsamem Tempern bei 400—600°C einen neuen Träger, auf dem das Palladium niedergeschlagen werden kann.

Der Palladiumgehalt des Katalysators, bezogen auf das Trägermaterial, liegt gewöhnlich zwischen 0,05 bis 15 Gew.-%.

Das Gewichtsverhältnis der Zusätze zum Palladium kann unterschiedlich sein; es kann z. B. zwischen 10 000 : 1 bis 1 : 50, bevorzugt bei 100 : 1 bis 1 : 50 liegen. Man verwendet den Katalysator beispielsweise in Form von Strängen mit einem Durchmesser von 3 mm und einer Länge von 10 mm oder als Pulver, je nach dem vorgesehenen Anwendungszweck.

Cyclohexylamin und substituierte Cyclohexylamine finden Verwendung auf dem Textilhilfsmittelsektor, Pflanzenschutzmittelsektor, als Korrosionsschutzmittel und als Monomere für Kunststoffe (Ullmanns Encyklopädie der techn. Chemie, 3. Auflage, Band 5, Seiten 681—685).

Herstellmöglichkeiten für den Katalysator

a) Auf strang- oder pulverförmiges γ-Aluminiumoxid wird das Edelmetall zusammen mit dem Zusatz (Mangan, Zink, Silber, Seltenerdmetalle u. a.) in der gewünschten Menge durch Tränken mit beispielsweise deren Nitratlösungen und anschließendes Eindampfen bis zur Trockne aufgebracht. Danach wird 6 Stunden bei 550°C getempert und im Wasserstoffstrom bei 300°C reduziert.
b) Auf strang- oder pulverförmiges γ-Aluminiumoxid wird zunächst die gewünschte Menge des Zusatzes (Mangan, Zink, Cobalt, Magnesium, Lithium u. a.) durch Tränken mit entsprechenden wäßrigen Nitrat- oder Formiatlösungen aufgebracht und bei 150°C getrocknet. Der derart vorbehandelte Träger wird nun entweder 6 Stunden lang auf 550°C erhitzt oder, wenn Spinellbildung erreicht werden soll, 6 Stunden lang bei 1050°C geglüht. Nun wird der Träger mit wäßriger Edelmetallnitratlösung getränkt und durch 7stündiges Erhitzen bei 300°C im Wasserstoffstrom reduziert. Wurde zum Tränken eine Lösung eines Chlorids genommen, wird mit alkalischer Formalinlösung reduziert.
c) γ-Aluminiumoxid und ein basisches Oxid (MgO, CaO) werden in dem gewünschten Mengenverhältnis intensiv miteinander vermischt. Diese Mischung wird 6 Stunden lang auf 450°C erhitzt, anschließend mit Edelmetallnitratlösung getränkt und 7 Stunden mit Wasserstoff bei 300°C reduziert. Der Katalysator wird mit einer 5%igen wäßrigen Hydrazinhydratlösung nachreduziert und dann bei 120°C getrocknet.

Die in den nachfolgenden Beispielen angegebenen Siedepunkte beziehen sich, sofern nicht anders angegeben, auf atmosphärischen Druck, sonst auf verminderten Druck in mbar.

### Beispiel 1

In einem 300 ml fassenden Rührautoklaven wird eine Mischung aus 54 g 2,3,6-Trimethylanilin, 41 g flüssigem Ammoniak und 6 g Katalysator der Zusammensetzung 10 Gew.-% Palladium, 5 Gew.-% Praseodymoxid auf Aluminiumoxid bei 230°C und 250 bar Wasserstoffdruck bis zur Druckkonstanz hydriert. Das Reaktionsprodukt besteht aus reinem 2,3,6-Trimethylcyclohexylamin ($Kp_{17}$ = 78 bis 81°C).

### Beispiel 2

In einem 10-l-Rührautoklaven werden 1,7 kg 2,6-Dimethylanilin und 170 g eines pulverförmigen Katalysators, der 5,0 Gew.-% Palladium, 2,5 Gew.-% Psaseodymoxid auf Aluminiumoxid enthält, eingefüllt. Der Autoklav wird verschlossen und 1,4 kg Ammoniak aufgepreßt. Nun wird auf 230°C erhitzt und mit Wasserstoff ein Druck von 250 bar eingestellt. Man hält so lange auf Reaktionstemperatur, bis ein konstanter Druck erreicht ist (nach ca. 2 Stunden). Anschließend läßt man abkühlen, filtriert und erhält 1,75 kg rohes 2,6-Dimethylcyclohexylamin (Gemisch aus 3 stereoisomeren 2,6-Dimethylcyclohexylaminen). Man destilliert und erhält 1,72 kg reines 2,6-Dimethylcyclohexylamin, Kp = 167 bis 168°C, entsprechend einer Ausbeute von 97% der Theorie.

### Beispiel 3

Man verfährt analog Beispiel 2, verwendet aber einen Katalysator, der 10 Gew.-% Palladium, 0,11 Gew.-% Zink und 0,10 Gew.-% Cadmium auf Aluminiumoxid enthält. Ausgehend von Anilin wird Cyclohexylamin (Kp = 134°C) als Reaktionsprodukt erhalten, die Ausbeute beträgt 94% der Theorie.

### Beispiel 4

Man verfährt analog Beispiel 2, verwendet aber einen Katalysator, der 2,0 Gew.-% Palladium auf Lithium-Aluminiumspinell enthält. Ausgehend von 2-Methyl-6-tert.-butyl-anilin erhält man als Reaktionsprodukt 2-Methyl-6-tert.-butyl-cyclohexylamin (Kp = 220 bis 221°C) in einer Ausbeute von 95% der Theorie.

### Beispiel 5

Man verfährt analog Beispiel 2, verwendet aber einen Katalysator, der 10 Gew.-% Palladium, 5 Gew.-% Silber und 1 Gew.-% Mangan auf Aluminiumoxid enthält. Ausgehend von m-(4-Methyltetrahydropyran-2-yl)-anilin erhält man als Reaktionsprodukt 3-(4'-Methyl-tetrahydropyran-2'-yl)-cyclohexylamin (Kp = 80 bis 81°C/0,13 mbar) in einer Ausbeute von 95% der Theorie.

Beispiel 6

Man verfährt analog Beispiel 2, verwendet aber einen Katalysator, der 2 Gew.-% Palladium auf Zink-Aluminiumspinell enthält. Ausgehend von 2,6-Di-isopropyl-anilin erhält man als Reaktionsprodukt 2,6-Diisopropyl-cyclohexylamin (Kp = 239 bis 240°C) in einer Ausbeute von 92% der Theorie.

Beispiel 7

Man verfährt analog Beispiel 2, verwendet aber einen Katalysator, der 2 Gew.-% Palladium auf Cobalt-Aluminiumspinell enthält. Ausgehend von 2,2-Bis-(4'-aminophenyl)-propan erhält man als Reaktionsprodukt 2,2-Bis-(4-aminocyclohexyl)-propan (Dihexylan, Kp = 144 bis 147°C/0,4 mbar) in einer Ausbeute von 95% der Theorie.

Beispiel 8

In ein druckfestes zylindrisches Rohr mit einem Volumen von 1 l als Reaktor wird ein Katalysator in Form von Strängen (3 mm Durchmesser, 10 mm Länge), der 0,5 Gew.-% Palladium auf Magnesium-Aluminiumspinell enthält, eingefüllt und auf 230°C erhitzt. Bei dieser Temperatur wird über diesen Katalysator bei einem Druck von 250 bar stündlich eine Mischung aus 50 g 2,6-Dimethylanilin und 600 g flüssigem Ammoniak geführt. Gleichzeitig werden 200 Nl Wasserstoff/Stunde durch das Reaktionsrohr hindurchgeleitet. Das abfließende Produkt wird unter Druck abgekühlt und dann entspannt. Es fallen hierbei stündlich ca. 52 g Rohprodukt an. Man destilliert und erhält 51 g reines 2,6-Dimethylcyclohexylamin, entsprechend einer Ausbeute von 98% der Theorie. Zur kontinuierlichen Verfahrensgestaltung werden unverbrauchter Wasserstoff und Ammoniak im Kreis geführt.

Beispiel 9

Man verfährt analog Beispiel 8, verwendet aber einen Katalysator, der 2 Gew.-% Palladium auf einer Mischung aus 19,4 Gew.-% Calciumoxid und 80,6 Gew.-% Aluminiumoxid enthält. Ausgehend von 2,3,6-Trimethylanilin erhält man als Reaktionsprodukt 2,3,6-Trimethylcyclohexylamin (Kp = 78 bis 81°C/23 mbar) in einer Ausbeute von 98% der Theorie.

**Patentansprüche**

1. Verfahren zur Herstellung von cycloaliphatischen Aminen durch Hydrierung von aromatischen Aminen in Gegenwart eines Hydrierkatalysators, dadurch gekennzeichnet, daß man einen Trägerkatalysator verwendet, der als Träger Aluminiumoxid oder eine Aluminiumoxid enthaltende Verbindung, Palladium und eine basische Verbindung und/oder ein Element der Gruppe 1 B, 2 B, 7 B, Eisen, Kobalt oder Nickel enthält und daß man die Hydrierung in Gegenwart von Ammoniak bei einem Druck von wenigstens 100 bar durchführt.

2. Verfahren gemäß Anspruch 1, dadurch gekennzeichnet, daß der Katalysator als basischen Stoff ein Oxid, Hydroxid oder Carbonat eines Alkalimetalls oder eines Erdalkalimetalls enthält.

3. Verfahren gemäß Anspruch 1, dadurch gekennzeichnet, daß der Katalysator als basischen Stoff ein Oxid, Hydroxid oder Carbonat eines Seltenerdmetalls enthält.

4. Verfahren nach Anspruch 1, dadurch gekennzeichnet, daß der Katalysator eine Magnesiumverbindung enthält.

5. Verfahren nach Anspruch 1, dadurch gekennzeichnet, daß der Katalysator eine Praseodym/Neodym- oder Cerverbindung enthält.

**Claims**

1. A process for the preparation of a cycloaliphatic amine by hydrogenating an aromatic amine in the presence of a hydrogenation catalyst, wherein a supported catalyst is used which comprises, as carrier, aluminium oxide or an aluminium oxide-containing compound, palladium and a basic compound and/or an element of group I B, II B or VII B, iron, cobalt or nickel, and the hydrogenation is carried out in the presence of ammonia under a pressure of at least 100 bar.

2. A process as claimed in claim 1, wherein the catalyst contains, as basic compound, an oxide, hydroxide or carbonate of an alkali metal or alkaline earth metal.

3. A process as claimed in claim 1, wherein the catalyst contains, as basic compound, an oxide, hydroxide or carbonate of a rare-earth metal.

4. A process as claimed in claim 1, wherein the catalyst contains a magnesium compound.

5. A process as claimed in claim 1, wherein the catalyst contains a praseodymium/neodymium compound or a cerium compound.

## Revendications

1. Procédé pour la préparation d'amines cycloaliphatiques par hydrogénation d'amines aromatiques en présence d'un catalyseur d'hydrogénation, caractérisé en ce qu'on utilise un catalyseur sur support qui contient de l'oxyde d'aluminium ou un composé contenant de l'oxyde d'aluminium comme support, du palladium et un composé basique et/ou un élément des groupes 1 B, 2 B, 7 B, du fer, du cobalt ou du nickel, et en ce qu'on effectue l'hydrogénation en présence d'ammoniac sous une pression de 100 bars au moins.

2. Procédé selon la revendication 1, caractérisé en ce que le catalyseur contient, en tant que substance basique, un oxyde, un hydroxyde ou un carbonate d'un métal alcalin ou d'un métal alcalino-terreux.

3. Procédé selon la revendication 1, caractérisé en ce que le catalyseur contient, en tant que substance basique, un oxyde, un hydroxyde ou un carbonate d'un métal des terres rares.

4. Procédé selon la revendication 1, caractérisé en ce que le catalyseur contient un composé du magnésium.

5. Procédé selon la revendication 1, caractérisé en ce que le catalyseur contient un composé du praséodyme/néodyme ou du cérium.